Europäisches Patentamt

European Patent Office

Office européen des brevets

(19)

(11) EP 1 086 703 A2

(12) EUROPEAN PATENT APPLICATION

(43) Date of publication:
28.03.2001 Bulletin 2001/13

(51) Int Cl.$^7$: A61K 38/47

(21) Application number: 00124590.1

(22) Date of filing: 13.01.1996

(84) Designated Contracting States:
AT BE CH DE DK ES FR GR IE IT LI LU MC NL PT SE
Designated Extension States:
LT LV SI

(30) Priority: 13.01.1995 EP 95100446
07.07.1995 EP 95110638

(62) Document number(s) of the earlier application(s) in accordance with Art. 76 EPC:
96900321.9 / 0 804 227

(71) Applicant: NIKA HEALTH PRODUCTS LIMITED
FL-9490 Vaduz (LI)

(72) Inventor: Kiczka, Witold
Princeton, NJ 08540 (US)

(74) Representative: Büchel, Kurt F., Dr. et al
Büchel, Kaminski & Partner,
Letzanaweg 25
9495 Triesen (LI)

Remarks:
This application was filed on 10 - 11 - 2000 as a divisional application to the application mentioned under INID code 62.

(54) New applications of lysozyme dimer

(57) The present invention relates to pharmaceutical compositions containing a lysozyme dimer, preferably of high purity, i.e. with about 10 wt.% or less of unintended by-products, and new applications thereof. Such applications include topical, oral and parenteral administration of said compositions for non-specific immunostimulation as a measure of prevention and/or therapeutic treatment of human and animal diseases comprising cancer, hair growth disorders, fish diseases and bee diseases. The non-specific stimulation of the immune system is induced by a single or repeated application of the lysozyme dimer composition, preferably at concentrations of 5 to 500 µg/kg body weight.

Fig. 1

EP 1 086 703 A2

**Description**

TECHNICAL FIELD

The present invention relates to new applications of lysozyme dimer and to compositions containing such dimer. The new applications are based on a common principle of non-specific stimulation of the immune system and are particularly useful for the prevention and/or treatment of symptoms or diseases in connection with an impaired function of the natural defensive and regenerative systems in the human and animal body.

BACKGROUND OF THE INVENTION

In the late eighties it was discovered that the dimerized forms of certain enzymes, while substantially retaining the beneficial properties of the corresponding monomers, turned to be by far less toxic than the monomers and in some instances did not even display negative side effects at all when used in therapeutic doses. Antiviral and antibacterial compositions comprising as the active ingredient lysozyme dimer or other dimerized enzymes have been described in WO 89/11294. It is reported therein that lysozyme dimer is capable of inhibiting proliferation of a number of bacterial strains cultivated on samples taken from patients, when applied in concentrations of 1.25 - 20 mg/ml of the culture. It is also reported that the dimer is effective in treating canine parvovirus (CPV) infections when administered orally twice a day at a dose of 1 - 2 mg/kg of body weight. Later on, further attractive features of lysozyme dimers were found and additional therapeutical applications of the drug were developed, especially for the treatment of bacterial and viral infections as disclosed, for instance, in WO 94/01127.

In WO 94/01127 a model theory is presented that can help to understand the different effects observed with the lysozyme dimer. Although the entire mode of action of the lysozyme dimer is not yet fully understood it appears that there is additional curative capability that cannot be explained by the bacteriolytic activity of the corresponding monomer. The inventors observed certain immunostimulative effects of the dimerized lysozyme, particularly concerning the modulation of cytokine levels. Moreover, from their experiments they concluded that lysozyme dimer seems to prevent the penetration of bacterial cells by viruses, presumably by blocking certain regions of the outer cell surface and probably comprising virus receptor proteins.

The prior art discloses further results obtained in vitro with lysozyme dimer. Particularly, Bartholeyns and Zenebergh (Europ. J. Cancer, Vol.15, 1979, 85-91) tested dimerized lysozyme for cytostatic activities against liver cancer cells (HCT) *in vitro*. They observed a 73% ± 15% inhibition of cancer cell multiplication in the cell culture (ibid., p.89, Table 2).

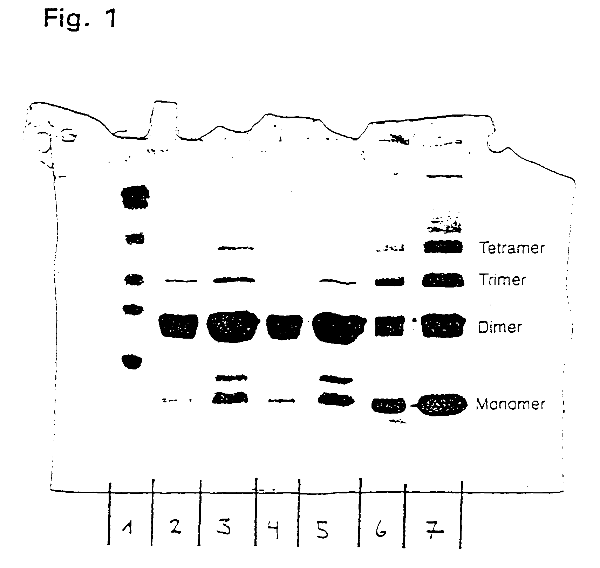

Surprisingly, except for WO 94/01127, no *in vivo* experiments with lysozyme dimer are reported so far. It is very strange and astonishing and up to now waits for explanation why neither Bartholeyns and Zenebergh nor any other researcher resumed this subject to promote further development of a promising discovery to combat cancer. A comparative showing (Fig. 1) of the purity of lysozyme dimer produced according to the method of Sorrentino et al., Eur. J. Biochem. 124, 183-189 (1982) and of the lysozyme dimer preferably used in the present invention revealed at least one possible reason: high concentrations of by-products such as lysozyme monomer, trimer and tetramer are found in the preparation produced according to Sorrentino et al., whereas the product preferably used in the present invention is highly purified, i.e., contains the desired lysozyme dimer in amounts of up to 90% by weight of the total lysozyme fraction of the preparation. A process for the manufacture of such highly purified lysozyme dimer has been described in WO 91/10731.

This strongly supports the assumption that the purity of the prior art lysozyme dimer was simply not good enough for *in vivo* experiments and applications because it was known in the art already 15 years ago that the monomeric form of lysozyme, despite its beneficial antibacterial activity, is rather toxic and can cause inflammations and severe allergies and even toxic shock symptoms.

In light of such circumstances it appears more understandable why no competent researcher including Bartholeyns and Zenebergh - although recommending lysozyme dimer as a promising candidate for further investigations - has carried out further experiments during the past ten to fifteen years to develop lysozyme dimer applications *in vivo*.

In spite of such lack of research activities of the scientific world possibly due to a prejudice of the art against the use of lysozyme dimer *in vivo*, the present inventors carried out further research and developmental work to improve the method of production and purification of the dimerized lysozyme and to find *in vivo* human and animal applications for the product which led, for instance, to the antiviral and antibacterial applications disclosed in WO 94/01127.

Moreover, based on their knowledge of low toxicity of dimerized lysozyme compared to the monomer and on the availability of a new, highly purified lysozyme dimer preparation the present inventors attempted and started anti-cancer trials with lysozyme dimer preparations *in vivo,* although the prior art did not suggest its use to treat diseases other than bacterial or viral infections. It is one of the biggest advantages of the present invention to provide for the use of a highly purified lysozyme dimer for the manufacture of a pharmaceutical composition to supplement or replace the extremely toxic anti-cancer drugs usually applied in conventional chemotherapy.

BRIEF DESCRIPTION OF THE DRAWINGS

Fig.1 displays a comparative showing of the purity of lysozyme dimer preparations manufactured according to two different prior art techniques.

DETAILED DESCRIPTION OF THE INVENTION

The highly purified lysozyme dimer referred to in the present invention can be used for the manufacture of pharmaceutical compositions directly applicable for the treatment of animals and humans due to its low amount of toxic monomer.

As indicated above, such purified lysozyme dimer contains about 10% wt. or less of unintended by-products, and can be obtained via dimerization of lysozyme monomers of any origin, e.g. of lysozyme monomers derived from humans, animals, eggs, plants, microorganisms, the monomers being either naturally isolated in native form or manufactured via chemical or genetic engineering methods to yield lysozyme monomers of the same or essentially the same chemical and biological nature as the naturally occurring ones.

In tests on *in vitro* cell cultures and *in vivo* test models, preferably mice and rat test models, the inventors of the present invention could successfully demonstrate that the applied lysozyme dimer compositions display remarkable potency in the inhibition or even total prevention of cell proliferation of cancerous tissues *in vitro* and *in vivo*. Such curative action could be confirmed for various types of cancer, including but not restricted to those that are known in the art to be induced or affected by viruses. These viruses comprise in particular the human papilloma virus, the Epstein-Barr virus, hepatitis B virus and retroviruses such as HTLV-I (adult T-cell leukaemia virus), HTLV-II (lymphoma virus) and HIV-1 (human immunodeficiency virus) including subtypes.

The types of cancer induced or affected by the action of these viruses comprise i.e. cervix carcinoma, carcinoma of the urogenital tract, Kaposi sarcoma, primary liver cell carcinoma, leukaemia, T-cell lymphoma, AIDS, or any subtype thereof. It is an object of the present invention to provide for a pharmaceutical composition and a method to prevent and/or treat cancer in animals and humans.

Another beneficial application of the highly purified lysozyme dimer was surprisingly discovered in clinical trials when lysozyme dimer compositions were administered to patients with more or less infected postoperative wounds, e.g. after amputation of a lower extremity. It turned out that the local application of the dimer not only successfully defeated the wound infection but also stimulated hair growth and hair renewal within the treated area. This surprising discovery led to further investigations and finally to the manufacture of pharmaceutical compositions applicable in cases of hair growth disorders, particularly hair growth disorders based on immunological malfunctions or dysfunctions such as, e.g., in case of alopecia areata.

Such compositions can at least partly fulfill the criteria of a long desired composition acting as a hair growth stimulant. The underlying principle of this completely unexpected effect is believed to comprise amongst others the stimulation and improvement of immune defense mechanisms, improvement of the blood circulation in the superficial tissue layers and the skin, general non-specific improvement of immunological functions and, probably, some more still unidentified effects.

Another surprising and commercially very interesting application for a pharmaceutical composition containing lysozyme dimer has been found in fish farming and bee-keeping when in a screening experiment conventional antibiotics have been replaced with different non-specific immunostimulants including also the lysozyme dimer.

The use of immunostimulants in fish culture for the prevention and/or therapy of fish diseases is a promising new development (Siwicki and Anderson 1990; Siwicki et al. 1994). At present, substances for treating fish diseases include antibiotics, drugs and chemicals used for sterilizing fish-holding ponds (Stoskopf, 1993). While each therapy is at least partially effective in the treatment of a particular disease, problems arise with the accumulation of these substances in the environment and in the meat of the fish as well as with the emergence of pathogenic strains of microorganisms resistant against various antibiotics.

In case of bee-keeping it is a widespread practice to kill colonies of bees if there is evidence that the bees are affected by microbial, e.g., bacterial and/or viral infections. Such practice avoids spreading the infections among other colonies and thereby minimizes the losses. There is therefore a need to provide an alternative and reliable method for the prophylactic and/or therapeutic treatment of honey-bees that combines both the advantage of an antibiotic additionally strengthening the immune system of bees and an antibiotic that will not result in the development of antibiotic-resistant microbial strains. The present invention provides for a method to meet such need. Moreover, even if lysozyme dimer was present in the final honey product in detectable concentrations this would likely be regarded as an improvement of quality rather than as a change to the worse, due to the benefit of the non-specific immunomostimulative effect of lysozyme dimer.

In general, immunostimulants comprise a group of biological and synthetic compounds that enhance the non-specific cellular and humoral defense mechanisms in animals and humans. Immunostimulants such as beta-glucan, chitosan, levamisole, trace mineral and mineral combinations, and various products derived from many plant and animal sources are effective in preventing diseases. Several types of betaglucans seem to be especially promising for the stimulation of the cellular and humoral non-specific immune response of fish.

The non-specific defense mechanisms which include phagocytosis and the production of oxidative radicals are quickly activated by the immunostimulants and are rapidly prepared to protect the fish against pathogenic microorganisms such as, for example, viruses, bacteria, mycoplasms and funghi, and/or against parasites or other pathogenic agents. Thus, these mechanisms are superior to the specific immune response that requires a longer period of time for the development of a specifically adapted immune response including antibody build-up and specific cellular activation.

The very encouraging results obtained in the first *in vivo* trials, indicating significant improvement of immunological defense mechanisms, have led to launching a detailed research program on the effect of lysozyme dimer on the specific and non-specific cellular and humoral defense mechanisms of fish, particularly of trout and salmon, and of honey-bees.

Therefore, it is an object of the present invention to provide for novel applications of lysozyme dimer comprising its use for the manufacture of a pharmaceutical composition for the stimulation of a non-specific cellular and humoral immune response in fish and honey-bees.

It is another object of the present invention to provide for a method of using pharmaceutical compositions containing said lysozyme dimer to induce a non-specific stimulation of the immune system by a single or repeated application of the composition, for the prevention and/or therapy of naturally occurring diseases of fish and bees.

*In vivo* experiments have shown that in some cases, e.g., where the fish are readily impaired by certain chemicals or undesired bioactive substances such as by-products of pharmaceuticals administered to them, it might be preferable to treat the fish with the highly purified lysozyme dimer reported in W091/10731 which contains about 10% by weight or less of unintended by-products and which is essentially free from the monomeric form of lysozyme. In order to reduce breeding costs in fish farming and/or in bee-keeping it might, however, also be acceptable to administer a lysozyme dimer preparation of lower purity as long as it is applied at a dose that does not cause adverse effects due to the presence of toxic by-products, especially of lysozyme monomer.

The lysozyme dimer referred to herein can either directly be applied to the patients in need thereof or can be used for the manufacture of pharmaceutical compositions to be applied in usual galenic forms. Gels, ointments, or liquid compositions comprise the lysozyme dimer preferably in a concentration of about 0.01 - 10 mg/ml and frequently in a concentration of about 0.1-1.0 mg/ml. They are usually prepared as sterile and apyrogenic compositions and optionally further comprise at least one physiologically acceptable solvent and/or carrier and/or at least one suitable preservative.

The pharmaceutical compositions containing lysozyme dimer are useful and intended primarily for topical and/or parenteral application comprising local injection, e.g., in the vicinity of a solid tumor, or external applications on the surface of the body including subcutaneous injection. Intravenous injections may replace or additionally support topical applications in the course of a therapy. It has, however, also proven very efficient to administer lysozyme dimer compositions to mucosal membranes, preferably via inhalation (nasal, mouth, pharyngeal mucosa) of liquid compositions or topical application (e.g. vaginal, cervical mucosa) of liquid or creamy compositions or tampons impregnated with lysozyme dimer material.

In some cases it is preferred to apply the lysozyme dimer orally, preferably in usual galenic forms such as for instance tablets, capsules or dragees or in the form of pellets, granules, flocs or as a powder. These solid compositions frequently contain the active drug in an amount of about 0.01 - 10 mg, preferably about 0.01-1.0 mg per g of the total composition. It is also preferred that they further comprise at least one suitable carrier and/or preservative and/or other usual additives such as for instance a flavor or a colorant. In case of treating fish solid compositions may be added to the nutrients. Alternatively or additionally, the lysozyme dimer may be dissolved in the water of the fish-holding pond because it is believed that the active drug may also be resorbed via the gills of the fish. In case of bee-keeping lysozyme dimer compositions can be applied by dissolving solid compositions in drinking water, tea, sugar solutions or other usual liquids prepared for the bees, e.g. as a feed substitute. It might, however, also be prepared as a - preferably concentrated - aqueous solution and mixed with honey, in order to ensure uptake of the drug by the bees.

A composition for oral use may, however, also be in the form of an osmotic system. In certain cases such as, for instance, in order to prevent and/or treat cancer of the urogenital tract, the local application of antiseptic dressings or tampons impregnated with an effective dose of the highly purified lysozyme dimer can be useful and beneficial.

The various types of the above mentioned lysozyme dimer compositions are preferably administered in a single or repeatable dose of about 0.005 to 0.5 mg/kg of body weight, especially at a dose of about 0.01 to 0.1 mg/kg of body weight. It goes without saying that the required concentration of active lysozyme dimer in the final pharmaceutical composition depends primarily on the size of the human or animal patient and of the kind of therapy or prophylactic treatment scheduled for the concerned patient. In most cases, however, the above mentioned concentration ranges are sufficient for a proper treatment. Nevertheless, it might become necessary, particularly in veterinary medicine, to increase the actual lysozyme dimer concentrations in the composition to a value beyond 10 mg/ml and below the solubility product of the dimer in the respective solvent, i.e. to about 20 mg/ml of liquid or ointment. In doing so, the volume of the composition to be administered can be kept a reasonable minimum for the ease of handling.

If possible, a combined administration protocol of the aforementioned compositions is preferred over a single therapy of either oral, parenteral or topical application.

Due to the essentially untoxic character of the highly purified lysozyme dimer the compositions containing such dimer may be administered over a long period of time, i.e., months or even years without causing harmful side effects. The time intervals for prophylactic or therapeutic administration of the drug may typically range from one or more times daily to weekly and monthly dosages and may also comprise even longer intervals, depending on the respective patient and the urgency of treatment as well as on the efficacy of the immunostimulation by the lysozyme dimer.

As indicated above, the present invention was at least partly made possible through the availability of high grade lysozyme dimer. The striking difference of product quality and, in particular, of the undesired lysozyme monomer share, is demonstrated by Fig. 1:

Lane 1 represents LMW prestained protein standards: Phosphorylase B 142.000 dalton, BSA 97.000, ovalbumin 50.000, carbonic anhydrase 35.100, soybean trypsin inhibitor 29.700, lysozyme 21.900 (Biorad, USA);

lanes 2 and 3 show purified lysozyme dimer LYDIUM KLP®602 (KLP-602), lot 506449, laboratory control; lane 2 loaded with 6.6 µg and lane 3 with 19.8 µg;

lanes 4 and 5 show another batch of KLP-602, lane 4 loaded with 6.6 µg and lane 5 with 19.8 µg;

lanes 6 and 7 show a lysozyme dimer preparation (KIW-607) manufactured according to Sorrentino et al., Eur. J. Bioch. 124, 183-189 (1982); lane 6 loaded with 6.6 µg and lane 7 with 19.8 µg.

The purified lysozyme dimer preparation KLP-602 contains four times more dimer than the compared product KIW-607, whereas the compared product KIW-607 contains six times more monomer than KLP-602.

In order that the invention described herein may be more fully understood, the following examples are set forth. It should be understood that these examples are for illustrative purposes only and are not to be construed as limiting this invention in any respect.

Example 1: Lysozyme dimer (KLP-602) in the treatment of lymphatic leukemia in AKR mice.

Materials and methods

Seven months old mice of both sexes of leukemic strain AKR, inoculated with Graffi virus from the Inbreeding Animal Center, Institute of Immunology and Experimental Therapy, Polish Academy of Sciences, Wroclaw, were used in the study. In AKR mice early activation of endogenous leukemic virus takes place, which leads to clinical manifestations of lymphatic leukemia at the age of seven to nine months. The mice were divided into three groups of 30 animals each:

Group I (experimental)

- animals were given KLP-602 subcutaneously once at a dose of 20 µg/kg in 0.3 cm$^3$ PBS;

Group II (experimental)

- animals received KLP-602 twice, at the same dose as above;

Group III (control)

- animals received 0.3 cm$^3$ PBS twice.

The mice were killed by bleeding three and six weeks after the last KLP-602 injection (10 mice from each group). Ten mice from each group were left alive.

The following examinations were performed:

1. Measurement of the total body weight and the mass of the internal organs such as liver, spleen, thymus and external cervical lymph nodes, with the estimation of the organ mass to total body weight ratio. The therapeutic activity ratio was evaluated using the formula L/K x 100 %, where L is the mean survival time of the mice treated with KLP-602 and K is the mean survival time of the controls.

2. Histopathological examinations of the biopsies of the liver, spleen, thymus and lymph nodes. Sections were stained with H+E of the Gomori method for reticular fibers.

3. The level of free radicals in the serum was estimated using the chemiluminescence (Chl) method which allows detection of very weak photon luminescence of the serum.

4. Phagocytic activity of the polymorphonuclears (PMN) was estimated using NBT and chemiluminescence (Chl) tests.

Results:

Single (Group I) and double (Group II) subcutaneous administration of KLP-602 to the highly leukemic AKR mouse strain, at a dose of 20 μg/kg, caused a statistically insignificant increase of body weight, and a decrease of the mass of internal organs such as: spleen, thymus, lymph nodes and liver (the last one was statistically insignificant) in comparison with the control animals (Group III) measured after three weeks of study (Table 1).

Increase of total body weight in both treated groups, decrease of the mass of the spleen, thymus and lymph nodes in Group I, and the increase of liver and thymus in Group II, when compared with the controls (Group III) occurred after six weeks of the study (Table 1).

Table 2 shows the ratio of the internal organs' weight to the total body weight. After three weeks the increase of thymus mass was observed in Group III (control) in comparison with both experimental groups (I and II). After six weeks, there was an increase of the mass of the spleen, thymus and lymph nodes in Groups II and III when compared with Group I.

The histopathological examinations conducted after three and six weeks revealed moderate (in the experimental groups) and excessive (in the control group) colonization of the cervical lymph nodes, thymus and spleen with lymphoid cells. However, the leukemic infiltrates in the liver were found only in the control. The lymphoid cells had oval or circular hyperchromatic nuclei with numerous nucleoli and atypical mitotic figures. These cells were accompanied by scanty low-differentiated centroblastic cells, reticulum cells and isles of proliferating megakaryocytes in the spleen. The stroma of the lymphatic organs such as the spleen, lymph nodes, and thymus contained scanty, thin reticulum fibers.

Chemiluminescence Examinations:

The activity of free radicals in the sera of the control mice was $K=18 \times 10^{-3}$ after three weeks of the study. In Group II the activity of free radicals was lower ($K=10 \times 10^{-3}$). After six weeks the free radicals level was $K=10 \times 10^{-3}$ in the control animals, lower in Group II ($K=16 \times 10^{-3}$), and slightly higher in Group I ($K=1.5 \times 10^{-3}$). The marked increase of the phagocytic activity of PMN was noted in both experimental groups as compared with the control group after three weeks and was: 4688 Chl (Group I), 3485 Chl (Group II), and only 2052 Chl for the control Group. After six weeks the phagocytic activity in Group II was equally high (3264 Chl) and it was lower in Group I (2477 Chl). A significant increase of phagocytic activity measured by a NBT test was noted after three weeks in both treated groups and was 0.27 and 0.26 in Groups I and II, respectively. After six weeks increase of phagocytic activity was lower and took place only in Group II. Phagocytic activity in the control group was 0.22.

The therapeutic efficacy index of KLP-602 was 128% and 150% in Groups I and II respectively, being above the minimal 125%. This indicates the marked immunostimulative properties of KLP-602 and qualifies it for further studies.

The leukemic infiltrates were considerably smaller in both experimental groups than in the control group and were even absent in some mice. The leukemic infiltrates were present in the thymus, spleen and lymph nodes. In addition, they were found in the liver of the control animals. The results of the study indicate that KLP-602 can partially inhibit or delay the progress of leukemia in AKR mice. To the contrary, the rapid enlargement of the lymphatic organs with morphological changes, typical for well differentiated lymphoid leukemia, occurred in the animals treated with the placebo.

Moreover, KLP-602 inhibits the increase of free radicals in mice serum. It inhibits free radical processes similar to the action of antioxidants such as vitamin E, C, or selenium. It is possible that the inhibition of the leukemic cell proliferation in AKR mice treated with KLP-602 depends on the inhibition of free radical induced processes. In control mice (without KLP-602) the level of free radicals was higher and correlated with the intensity of the disease.

KLP-602 also stimulates the phagocytic activity of the mononuclear phagocyte system (MPS) measured by two independent methods, NBT and Chl, and thus stimulates cell-mediated immunity. It can also modulate TNF synthesis. Moreover, the "tumor rejection antigens" present on the leukemic cell surface can be recognized by T-cells cooperating with MPS. The tumor anti-infiltrating lymphocytes (TIL), 50-100% more effective than lymphokine-activated killers (LAK), are among those T-cells. They have $CD_4$ or $CD_8$ or mixed phenotype and they express the IL-2 receptor on their surface. It is still unknown why they are paralyzed by neoplastic cells.

The reticulo-endothelial system with MPS cell stimulation is an essential immunological and antiviral mechanism. Stimulation of these cells results in interferon production and indirectly activates specific and non-specific immunological processes, B-cell, T-cell or NK dependent.

The MPS cells participate in immunological processes through their receptors such as Fc receptors, $C_3$ receptors or class II HLA receptors and also lectins, transferrins, urokinase, insulin and fifty other lesser-known receptors. Any changes of their expression and affinity indicate activation of the cells. MPS cells can phagocytize NO-sensitive cells because they can synthesise this substance. The activated phagocytes are called "angry macrophages" or "blood thirsty" cells. MPS cells can destroy viruses through their cytotoxic and cytolytic properties, defective virion production and interferon synthesis.

The promising results of the preliminary study on AKR mice indicate that KLP-602 can successfully be used in the treatment of leukemia in AKR mice.

Table 1.

| Total body mass (g) and mass of internal organs (mg) in control and experimental mice ($\bar{x} \mp S$; upper values indicate S, lower values indicate x) | | | | | | |
|---|---|---|---|---|---|---|
| | Three Weeks | | | Six Weeks | | |
| Group | I | II | III | I | II | III |
| Total Body Mass | 25.27 | 26.20 | 23.96 | 26.77 | 27.30 | 25.57 |
| | 2.22 | 1.67 | 1.23 | 1 .10 | 2.34 | 1.68 |
| Spleen | 0.07 | 0.08 | 0.09 | 0.07 | 0.23 | 0.22 |
| | 0.00 | 0.02 | 0.01 | 0.00 | 0.01 | 0.00 |
| Thymus | 0.06 | 0.07 | 0.08 | 0.08 | 0.21 | 0.15 |
| | 0.01 | 0.02 | 0.02 | 0.01 | 0.03 | 0.07 |
| Lymph Nodes | 0.04 | 0.04 | 0.05 | 0.04 | 0.07 | 0.08 |
| | 0.00 | 0.00 | 0.01 | 0.00 | 0.04 | 0.04 |
| Liver | 1.38 | 1.36 | 1.43 | 1.61 | 1.89 | 1.67 |
| | 0.15 | 0.38 | 0.05 | 0.18 | 0.42 | 0.15 |

Table 2.

| Internal organs' mass to total body mass ratio in control and experimental mice (%) | | | | | | |
|---|---|---|---|---|---|---|
| | Three Weeks | | | Six Weeks | | |
| Group | I | II | III | I | II | III |
| Spleen | 0.30 | 0.31 | 0.37 | 0.29 | 0.84 | 0.87 |
| Thymus | 0.23 | 0.26 | 0.33 | 0.33 | 1.06 | 0.61 |
| Lymph Nodes | 0.18 | 0.18 | 0.21 | 0.15 | 0.26 | 0.26 |
| Liver | 5.49 | 5.25 | 5.81 | 6.02 | 6.35 | 6.40 |

Example 2: Effect of a highly purified lysozyme dimer preparation (KLP-602) on hair growth in alopecia areata.

The study was conducted to determine whether a balsam containing KLP-602 acts on hair growth in alopecia areata. In addition, the tolerability of this balsam when applied on the skin was estimated.

Materials and Methods:

The study was a controlled, comparative clinical study. Twelve subjects (two male and ten female patients) with alopecia areata in "telogen" stage in general good health condition were involved in the study. They were aged from 19 to 50 years. Twelve subjects (three male and nine female patients) with alopecia areata treated with MINOXIDIL® were considered as a control group. Their ages ranged from 19 to 49 years. The distribution of subjects by sex and age within two treatment groups was similar for the two groups.

Patient selection criteria:

Inclusion criteria:

    1. Alopecia areata in "telogen" stage;
    2. Age 18-50 years.

Exclusion criteria:

    1. Alopecia areata in "dystrophy" stage;
    2. Another type of alopecia;
    3. Any other skin disease.

Concomitant medication:

Concomitant medication was not allowed unless it was required for treating the general health conditions.

Assignment of treatment numbers:

During the study persons who fulfilled the selection criteria were numbered in tested groups in sequential order. The investigator entered the corresponding number in the appropriate place on each Case Report Form (CRF).

Trial medication:

The hair balsam manufactured by NIKA according to the original formula was provided in 100 ml identical plastic bottles with a calibrated small-tipped dropper.
A 0.05% KLP-602 lotion was applied on the scalp three times a day according to the instructions of the attending dermatologist. The treatment began immediately after recognizing alopecia areata by the dermatologist.
A 2% MINOXIDIL® solution was applied twice daily to affected skin areas.
The duration of the treatment was 16 weeks. After the treatment the patients were observed for 2 months of the follow up period.

Evaluation of safety

Subjects for both tested groups were screened by means of a full history and medical examination for entry and end of study. Blood and urine samples for routine laboratory test were taken before and after treatment. Laboratory studies included the following tests:

Hematology:

Hemoglobin, hematocrit, total and differential white cell count

Serum Biochemistry:

ALT (GPT), AST (GOT), alkaline phosphatase, bilirubin

Urine:

dipstick urine analysis for protein, blood and glucose.

Patients were seen monthly during 4 months of therapy and 2 months of follow up period. At each visit, the subjects were questioned about the presence of general or local adverse events such as irritation, burning, itching; treatment areas were examined for erythema or other local side effects.

Evaluation of efficacy:

Efficacy parameters

1. The primary efficacy parameter was the hair growth
2. The secondary efficacy parameter was healing time of the altered scalp.
3. Photographic documentation was taken at the following points in the study:

   before application of hair balm with KLP-602
   at the time of hair growth
   at the end of application hair balm with KLP-602

Microscopic evaluation (trichogram) was performed. Standardized trichograms on hair taken from the border of alopecia areata lesions reveal most useful information on the course and severity of the disease and also efficacy of therapeutic measures.

At each visit, the scalp was evaluated for signs of hair growth which was categorized as:

- none
- slight - visible regrowth (vellus or few scattered solitary terminal hairs)
- moderate - incomplete (partial)
- complete

"cosmetically acceptable" hair growth was defined as sufficient to cover the scalp and conceal areas of residual hair loss.

Results:

All the subjects enrolled to the study (12 treated with 0,05% KLP-602 and 12 from control group treated with 2% Minoxidil solution) completed a 4 months therapy period and 2 months follow up.

Efficacy

The most useful measure of efficacy was the investigators' assessments at the end of the study. Overall, eight of the twelve subjects (66%) in KLP-602 group and six of twelve subjects (50%) in the Minoxidil group showed increased hair growth during the study. Of these, four subjects in the KLP-602 group (33%) and three in the Minoxidil group (25%) were considered to have complete, cosmetically acceptable hair growth. Partial hair growth was seen in three from twelve assessable subjects in the KLP-602 group (25%) and three in the Minoxidil group respectively (25%). In one subject in the KLP-602 group (9%) minimum hair growth was observed. There was no hair growth in four of twelve (33%) in the KLP-602 group and in six of twelve (50%) in the Minoxidil group.

In the KLP-602 group with alopecia at an extent of less than 25% of the scalp, hair growth was obtained in eight out of eight subjects and in six out of seven in the Minoxidil group, respectively. No regrowth was observed in subjects with alopecia at an extent of more than 25% of the scalp in both treated groups.

The mean time of hair growth response in correlation with the duration of the treatment ranged from one until three months in both groups. Three subjects in the KLP-602 group responded after one month of treatment. In one female the hair growth after one month was with terminal hair of about 5 mm length and the bald scalp areas were covered with dense hair regrowth.

At the control visit after the second month this woman appeared with alopecia totalis (emotional stress - divorce) and she did not respond to the further treatment, completed the study with negative result. In one subject in the Minoxidil group hair regrowth was observed after one month of therapy. In four patients in the KLP-602 group hair regrowth was obtained after two months of treatment. After three months of the study hair regrowth was observed in two subjects in the KLP-602 group and in three of the Minoxidil group, respectively.

Safety

No general side effects were observed during the therapy and follow up period. No abnormalities in laboratory blood and urine values were noted at the baseline and at the end of the study in both treated groups.

Local adverse events observed during the study: One female in the KLP-602 group complained of burning and itching of the scalp in the second month of the treatment. She connected these symptoms with wearing of a new wig. The sensations disappeared within two weeks without any additional treatment, she did not stop the therapy and completed the study with good result. One subject treated with 2% Minoxidil solution complained of greasy hair, no significant clinical signs were observed in the examination of the scalp. The patient correlated this event with the trial but he was willing to continue the treatment.

No significant findings were noted in correlation of the hair regrowth and the age of patients, localization of bald areas and the duration of the current episode, which was short and similar for both groups and rated from one week to one year maximum. All non-responders in both treated groups had alopecia areata anamnesis lasting for at least five years (5 -15 years).

Discussion

The study was to evaluate the safety and efficacy of topically applied 0.05% KLP-602 lotion the hair growth in patients with alopecia areata and to compare it with the effectiveness of a 2% Minoxidil solution during a four months therapy.

The hair balsam containing KLP-602 was well tolerated by patients. 66% of subject treated with 0.05% KLP-602 lotion showed the increased hair growth during the study in comparison to 50% of subjects in Minoxidil group.

Cosmetically acceptable hair growth was seen in 33% of patients in KLP-602 group and in 25% in Minoxidil group respectively. Better treatment results were observed in patients with an extent of alopecia lower than 25% of the altered scalp. 0.05% KLP-602 lotion was easy to use topically and did not cause any local nor general adverse events. Considering the short time of the study (16 weeks) the results obtained are very encouraging.

Conclusions

1. The hair balm containing KLP-602 was well tolerated and did not cause any side effects.

2. Topical application of 0.05% KLP-602 was easy and safe.

3. No local or general side effects and no derives in laboratory values were observed during the study.

4. Due to short time of the therapy (4 months) and 2 months follow up, the complete assessment of the results and performance of the treatment was impossible, although this period was long enough to observe encouraging efficacy of treatment.

5. The results obtained in the trial showed that 0.05% KLP-602 lotion is comparable to a 2% Minoxidil solution and even more effective in alopecia areata treatment.

6. Preclinical investigations and the results obtained in the study demonstrate that KLP-602 applied topically induces hair growth with patients suffering from alopecia areata.

Summary

The study was performed to estimate efficacy and tolerability of a hair balsam containing KLP-602 on hair growth in alopecia areata. Applications of 0.05% KLP-602 lotion three times a day were generally well tolerated in the 4 months treatment period. No local or systemic side effects were observed. Better hair growth response was seen in the KLP-602 group than in the Minoxidil group. Hair regrowth response was observed in 66% of the subjects with 0.05% KLP-602 lotion. The percentage of patients who responded with moderate or dense hair regrowth was greater among the patients whose balding area involved less than 25% of the scalp. 0.05% KLP-602 seems to be a remarkable agent in alopecia areata treatment.

Example 3: Prophylactic application of lysozyme dimer to induce a non-specific stimulation of the immune system of fish

Test design

Three hundred healthy fish of the species rainbow trout *(Oncorhynchus mykiss,* family of salmonidae), weighing 90-100 g each, were subjected to the trial. Lysozyme dimer manufactured according to WO91/10731 (herein designated "KLP-602") containing about 10% by weight or less of unintended by-products and being essentially free from the monomeric form of lysozyme was dissolved in PBS to prepare injection solutions for the administration of 10 and 100 μg lysozyme dimer per kg body weight, suitable for intraperitoneal injection into fish. The drug was administered once a day. The therapeutical protocol comprised different groups of fish subjected to drug application either on day 1 only, on day 1 and day 3, or on days 1, 3 and 5, respectively. The fish of the control group were not subjected to lysozyme dimer administration.

After 1, 2, 3 and 4 weeks from the administration of the last injection 10 individuals each were sampled from each of the treated groups, and from the control. The fish were immobilized and about 2 ml blood were collected from the caudal vein by means of a heparinized vacuette (Greiner). The phagocytic ability of PMN and MN cells, the respiratory burst activity and potential killing activity of PMN and MN cells, the MPO activity in PMN phagocytes, and lysozyme and gamma-globulin levels in the serum were determined.

Also, 20 individuals of each group were subjected to a disease (furunculosis) challenge test. The fish were given single intraperitoneal injections of a bacterial suspension of *Aeromonas salmonicida* grown on nutrient broth for 48 hours. The mortality (death rate) of the animals was determined and its interrelationship with the applied bacterial infection was confirmed by the isolation of pathogens from the kidneys of dead animals.

Results

In the KLP-602 treated groups, all of the above mentioned immunological parameters were significantly increased (P<0.05) over the corresponding values of the control group. This surprising result was observed in all groups, i.e., after one-, two- or threefold application of KLP-602 and lasted for the entire observation period of up to 4 weeks after the last injection.

Also, the mortality of fish in the KLP-602 treated groups was significantly lower than in the control group. Respiratory burst activity was even considerably higher, as were the phagocytic indexes and neutrophil myeloperoxidase (MPO) levels of the groups treated by two- and threefold application of KLP-602.

The known susceptibility of rainbow trout to virulent bacterial challenge by *A. salmonicida* and the results observed with the groups prophylactically treated with the lysozyme dimer composition suggest that the drug in fact was able to induce a short-term protection against said microbial challenge. It was further recognized, that the protection was higher in the groups that received two or three doses of KLP-602 than in the groups treated by a single application only .

Conclusion:

The application of highly purified lysozyme dimer to fish enhances the non-specific defense mechanisms and significantly improves the protection against microbial challenge, e.g., by A. salmonicida (causing furunculosis). This observation is of a great importance for a prophylactic and therapeutic treatment of fish in the fish breeding industry in order to minimize the losses of fish due to frequently occurring fish diseases, particularly during the spring season.

Example 4: Therapeutic application of lysozyme dimer to induce a non-specific stimulation of the immune system of fish

Test design

Two hundred rainbow trouts were involved in this comparative study of non-specific defense mechanisms in the course of a naturally occurring infection with IPNV (Infectious Pancreatic Necrosis Virus) and upon application of a composition comprising dimerized lysozyme (KLP-602). After the identification of IPNV infection, part of the fish were treated with KLP-602, while a control group remained untreated. The major parameters characterizing the non-specific cellular and humoral defense mechanisms were analyzed by immunological and serological methods. These methods included the weekly determination of the total leukocyte number, relative leukocyte count, phagocytic ability of PMN and MN cells, respiratory burst activity and potential killing activity, MPO activity in neutrophils, lysozyme and ceruloplasmin activity in the plasma, and total protein and gamma-globulin levels, during an observation period of 2 months.

Replicated groups of rainbow trout were fed diets containing the non-specific immunostimulant KLP-602 at a dose of 20 μg per kg body weight. Lyophilized samples of KLP-602 were applied together with nutrients either orally in solid form (flocs, pellets) or dissolved in the water of the fish-holding pond, i.e., as an aqueous solution, for a period of seven days. It is assumed that the drug might also be resorbed by a pathway via the gills of the fish.

Thereafter, they were sampled and assayed for changes in non-specific cellular and humoral defense mechanisms in weekly intervals. Each time, 10 fish were randomly selected for the immunological assays and the identification of IPNV. In addition, all fish were observed daily for unusual behaviour, morphological changes and mortality.

Results

In fish naturally infected by IPNV an immunosuppressive effect could be observed. Throughout the entire observation time the parameters indicating the non-specific cellular and humoral defense mechanisms were significantly reduced, whereas one week after feeding the lysozyme dimer containing diet some parameters, e.g., ceruloplasmin level, respiratory burst activity, immunoglobulin level, and lysozyme activity were significantly increased (e.g., approx. 31 % increase of ceruloplasmin level; approx. 40 % increase of lysozyme activity; approx. 33 % increase of respiratory burst activity) when compared to the control group. After application of KLP-602 immunostimulating effects on the non-specific defense mechanisms were unambiguously identified. Also, the cumulative mortality was lowest in the groups fed with the KLP-602 containing diet.

Conclusion

Fish treated with a diet containing lysozyme dimer (KLP-602) showed advantageous changes of the non-specific immunological defense mechanisms. These changes correlated with reduced mortality, i.e., indicating protection against the naturally occurring infection by IPNV, which finding strongly recommends to further develop and optimize the strategy of prevention and/or therapy of fish diseases by administration of the lysozyme dimer as referred to herein.

**Claims**

1.   Use of a lysozyme dimer for the manufacture of a pharmaceutical composition for prophylactic and/or therapeutic treatment of a hair growth disorder.

2.   Use according to claim 1, wherein the hair growth disorder is alopecia, particularly alopecia areata.

3.   Use according to claim 1 or 2, wherein the pharmaceutical composition comprises the lysozyme dimer at a dose of about 0.005 - 0.5 mg/kg, preferably of about 0.01-0.1 mg/kg of body weight.

4.   Use according to any one of the preceding claims, wherein the pharmaceutical composition is for topical, oral and/or parenteral administration in a single or repeatable dosage.

5.   Use according to claim 4, wherein the pharmaceutical composition is in the form of a gel, an ointment or a liquid, comprising the lysozyme dimer in an amount of 0.01-10 mg/ml, preferably in an amount of about 0.01-1.0 mg/ml.

6.   Use according to claim 4 for oral administration, wherein the lysozyme dimer composition is in a solid form, particularly in the form of a tablet, capsule, dragee, powder, pellets, granules, or flocs, comprising the lysozyme dimer in an amount of 0.01-10 mg, preferably 0.1-1.0 mg per g of total composition.

## Fig. 1